# EUROPEAN PATENT SPECIFICATION

(11) **EP 1 988 814 B1**
(45) Date of publication and mention of the grant of the patent: **02.07.2014**
(21) Application number: 07752068.2
(22) Date of filing: 01.03.2007
(51) Int. Cl.: A61B 1/32, A61B 17/02

(54) **SURGICAL RETRACTOR FRAME SYSTEM**
CHIRURGISCHES RETRAKTORRAHMENSYSTEM
SYSTEME D'ARMATURE POUR ECARTEUR CHIRURGICAL

(30) Priority: 01.03.2006 US 777771 P
(43) Date of publication of application: 12.11.2008
(73) Proprietor: Boss Instruments, Ltd., Earlysville VA 22936 (US)
(72) Inventor: WOOSTER, Nicholas, H., Hutchinson, MN 55350 (US)
(74) Representative: Caldwell, Judith Margaret
(86) International application number: PCT/US2007/005342
(87) International publication number: WO 2007/103173

(56) References cited:
- US-A- 5 792 046
- US-A- 6 116 588
- US-A- 6 116 588
- US-A1- 2002 177 753
- US-A1- 2004 199 055
- US-A1- 2005 035 515
- US-B1- 6 645 141
- US-B1- 6 645 141

## Description

### CROSS-REFERENCE TO RELATED APPLICATIONS

This application is a continuation-in-part of U.S. Provisional Patent Application Serial No. 60/777,771, filed on March 1, 2006.

### FEDERALLY SPONSORED RESEARCH OR DEVELOPMENT

Not Applicable.

### TECHNICAL FIELD

The present invention relates generally to clamps, and more specifically to toggle clamps and their use in surgical retractor frame systems.

### BACKGROUND OF THE INVENTION

Surgical retractor assemblies are used to hold incisions open and keep anatomical structures out of the way so the surgeon can have unobstructed access to a surgical site. Many of today's surgical retractor assemblies use either threaded screws or cam devices to lock various members of retractor assemblies in position. While such systems according to the prior art individually provide a number of advantageous features, they nevertheless have certain limitations. For example, threaded screws or cam devices require large operator input force to generate a sufficient clamping force to hold a retractor in place. Furthermore, such devices do not provide tactile feedback to the operator to indicate when positive clamping has been achieved. And, most require a relatively large space envelope for operation. Moreover, most locking devices require the use of two hands to lock a retractor or other surgical instrument in place. The present invention seeks to overcome certain of these limitations and other drawbacks of the prior art, and to provide new features not heretofore available. A full discussion of the features and advantages of the present invention is deferred to the following detailed description, which proceeds with reference to the accompanying drawings.

### SUMMARY OF THE INVENTION

The present invention generally provides a surgical retractor frame system having a toggle clamp.

According to one embodiment, the surgical retractor frame system is comprised of a table post with an integral table post clamp that attaches to an operating room table; a coupling clamp that attaches a frame to the table post; a frameset clamp that attaches support arms and an extension beam of the frame together; a retractor clamp that clamps onto a retractor or other hardware and also clamps onto a support arm of the frame; and retractors or other hardware attached via the retractor clamp to the frame.

According to another embodiment, the table rail post of the surgical retractor frame system is secured to a surgical table to support the other elements of the surgical retractor system. Its jaws clamp on a table rail located at the perimeter of the surgical table. The table rail post is the anchor for the surgical retractor frame system.

According to another embodiment, the coupling clamp, frameset clamp, and retractor clamp each employ a toggle device comprising a control element, typically, an actuation lever. The lever operates in an over-center fashion and drives various configurations of links, slides, and/or other levers that make up the coupling clamp, frameset clamp, and/or retractor clamp. The coupling clamp, frameset clamp, and/or retractor clamp in turn drive locking members that directly or indirectly clamp surgical instruments to a support arm.

According to another embodiment, the coupling clamp, frameset clamp, and/or retractor clamp can generate greater clamping forces than today's surgical retraction assemblies without excessive operator effort. The over-center toggle locking mechanism of the coupling clamp, frameset clamp, and/or retractor clamp provides positive clamping and tactile feedback to the user. The design of the toggle locking members can be easily modified to obtain customized output motions and force profiles, unlike threaded screw or cam locking devices which are fixed by their initial design.

According to another embodiment, the toggle mechanism of the coupling clamp, frameset clamp, and/or retractor clamp can achieve desired motion outputs with relatively short input lever motions. This reduces the space envelope required to operate the coupling clamp, frameset clamp, and/or retractor clamp, allowing them to be used in tighter spaces and creating less interference with the surgical sterile field. Additionally, the toggle mechanism can be locked and unlocked by an operator with one hand.

Other features and advantages of the invention will be apparent from the following specification taken in conjunction with the following drawings.

### BRIEF DESCRIPTION OF THE DRAWINGS

To understand the present invention, it will now be described by way of example, with reference to the accompanying drawings in which:
Figure 1A is a perspective view of an embodiment of the surgical retractor frame system;
Figure 1B is a top view of an embodiment of a portion of the frame without the retractor clamp;
Figure 1C is a side view of the embodiment of Figure 1B;
Figure 2A is an embodiment of a table post with an integral table post clamp;
Figure 2B is an exploded view of an embodiment of a table post with an integral table post clamp;
Figure 2C is a cut-away view along a center-line of an embodiment of a table post with an integral table post clamp in an unlocked position;
Figure 2D is a cut-away view along a center-line of an embodiment of a table post with an integral table post clamp in a locked position;
Figure 2E is an exploded view of an alternate embodiment of the table post;
Figure 2F is a partial exploded view of the alternate embodiment of the table post of Figure 2E;
Figure 2G is a cut-away view along a center-line of the table post of Figure 2E in an unlocked position;
Figure 2H is a cut-away view along a center-line of the table post of Figure 2E in a locked position;
Figure 3A is an exploded view of an embodiment of a frameset clamp;
Figure 3B is a top view of an embodiment of the frameset clamp of Figure 3A;
Figure 3C is a front perspective view of an embodiment of the frameset clamp of Figure 3A;
Figure 3D is a left side view of an embodiment of the frameset clamp of Figure 3A;
Figure 3E is a front view of an embodiment of the frameset clamp of Figure 3A;
Figure 3F is an exploded view of an alternate embodiment of a frameset clamp;
Figure 3G is a partial cut-away side view of an embodiment of the frameset clamp of Figure 3F with one clamp in the closed position;
Figure 3H is a partial cut-away side view of an embodiment of the frameset clamp of Figure 3F with one clamp in the open position;
Figure 3I is a partial cut-away top view of an embodiment of the frameset clamp of Figure 3F;
Figure 3J is an assembled perspective view of the frameset clamp of Figure 3F;
Figure 4A is an exploded view of an embodiment of a coupling clamp;
Figure 4B is a side view of an embodiment of a coupling clamp;
Figure 4C is a perspective view of an embodiment of a coupling clamp;
Figure 4D is a cross-sectional view of an embodiment of a coupl ing clamp along a vertical axis in an open position;
Figure 4E is a cross-sectional view of an embodiment of a coupling clamp along a vertical axis in a closed position;
Figure 5A is an exploded view of an embodiment of a retractor clamp;
Figure 5B is a cross-sectional view of an embodiment of a retractor clamp in an open position;
Figure 5C is a perspective view of an embodiment of a retractor clamp in an open position;
Figure 5D is a side view of an embodiment of a retractor clamp in a closed position; and,
Figure 5E is a perspective view of an embodiment of a retractor clamp in a closed position.

### DETAILED DESCRIPTION

While this invention is susceptible of embodiments in many different forms, there is shown in the drawings and will herein be described in detail preferred embodiments of the invention with the understanding that the present disclosure is to be considered as an exemplification of the principles of the invention and is not intended to limit the broad aspect of the invention to the embodiments illustrated.

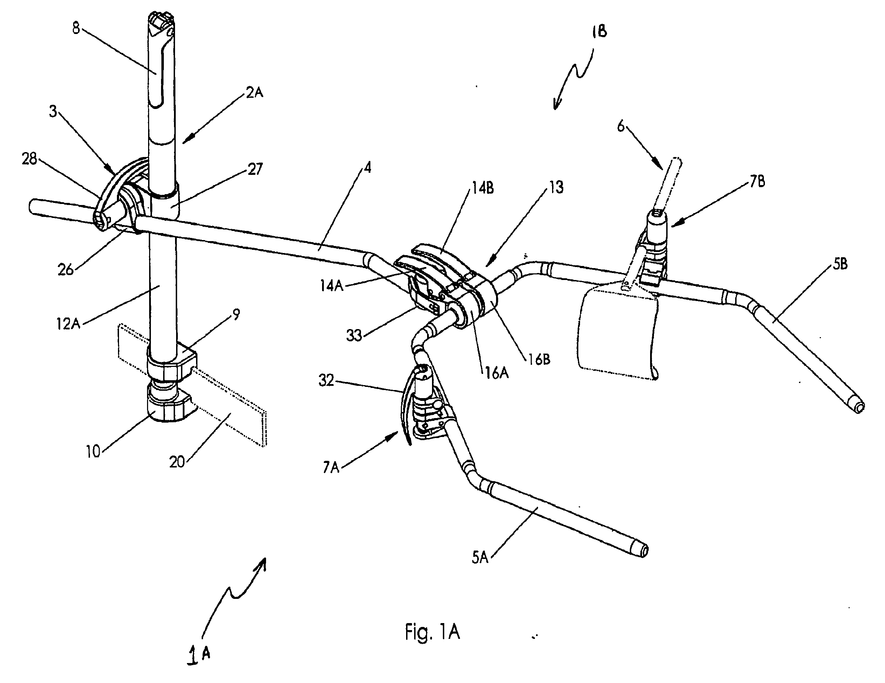

Referring now to the Figures, and specifically to FIG. 1A, there is shown a surgical retractor frame system 1A comprised of frame 1B, and table post 2A, with at least one toggle clamp connecting the various parts of the frame 1B or connecting the frame 1B to the table post 2A. Other embodiments or the present invention presented herein are a variety of toggle clamps, such as coupling clamp 3, table post 2A with an integral table post rail clamp 2B, retractor clamp 7A and frameset clamp 13. Frame 1B is comprised of a support arm 5, frameset clamp 13, extension beam 4, and retractor clamp 7A. Although the present invention may be practiced with extension beam 4 and support arm 5 manufactured as one piece, a preferred embodiment comprises extension beam 4 and support arm 5 as two separate pieces removably connected to one another.

Retractor clamp 7A is attached to a support arms 5A and/or 5B, if present, of frame 1B and used to hold a variety of surgical instruments, such as retractors. Retractors are often used to keep the surgical wound open. Although the present invention may be practiced with only one retractor clamp, a second retractor clamp 7B is preferably attached to support arm 5B of frame 1B with a typical retractor 6 in an orientation useful for holding open a surgical wound.

Frameset clamp 13 of frame 1B connects one or more support arms such as support arm 5A to frameset clamp 13 and connects extension beam 4 to frameset clamp 13. Coupling clamp 3 of surgical retractor frame system 1A couples extension beam 4 to table post 2A. Table post 2A with an integral table rail post clamp 2B anchors retractor frame system 1A to a rail 20 on an operating table.

In a preferred embodiment, the table post rail clamp 2B, coupling clamp 3, frameset clamp 13, and retractor clamp 7 all use a form of the toggle mechanism described in this specification. Preferably, they are all very similar to each other. Although other types of control elements may be used, for illustration purposes, the toggle mechanisms illustrated herein all have an actuation lever for driving various configurations of links and slides which in turn drive the locking members ofthe surgical retractor frame system 1A. Some ofthe benefits of the toggle mechanism are: (a) strong clamping forces are generated without excessive operator input; (b) the "over-the-center" toggle locking mechanism gives positive clamping and tactile feedback to the operator; (c) toggle mechanism output motion and force profiles can be modified by variation of linkage components: (d) desired motion outputs can be achieved with relatively short input lever motions, which reduces the space "envelope" required to operate the toggle mechanism for use in more compact spaces; and (e) in most applications the operator will be able to lock the toggle mechanism with one hand.

In one embodiment the toggle mechanism is driven by rotation of a lever or handle about a first pivot point (pivot 1). The lever is a link in the toggle mechanism. Preferably, it is a handle and provides leverage for the user to operate the toggle mechanism. The lever has a second pivot point (pivot 2), which is eccentric with respect to pivot 1, and rotates about pivot 1. Attached at, and rotating about pivot 2 is an intermediate link. The intermediate link pivots about pivot 2 on the lever and about a pivot point on an output link (pivot 3) which may either move along a line or pivot about another point. The intermediate link is manipulated or driven by rotation of the lever. Motion of the output link or output member can be varied by manipulating the distances between pivot points on the lever and the intermediate links. The pivot points are generally hinged connections.

In a preferred embodiment of the present invention, the lever is capable of locking in a closed position. It locks in position when pivot 2 passes "over center" through a line defined by pivot 1 and pivot 3. At the position where pivots 1, 2 and 3 are all in a line, compression of the clamp is greatest. By continuing to rotate the lever about pivot point 1, pivot point 3 is forced from being in the line causing a locking of the lever, intermediate link and output link. Forcing the pivot point 3 to be positioned away from the line joining the three pivot points is referred to as "passing over center". Thus, when pivot 3 passes over center in either direction, it cannot spontaneously pass over center in the other direction without the operator's assistance. Thus, when the lever is closed, it locks into position.

In various embodiments, one or more toggle clamps utilizing the toggle mechanism described above to provide the toggle clamping function for the surgical retractor frame system. Although specific examples are illustrated in the drawings and specification, there are many other attaching means well known in the art for attaching parts of a surgical retractor frame system together, such as welding, screwing parts together or utilizing clamps that are not toggle clamps. Although it is preferred to exclusively use toggle clamps to attach the parts that are shown attached in the Figures provided, the use of fewer or just one toggle clamp provides advantages, described above, over use of other attaching means. Accordingly, the surgical retractor frame system may be utilized with at least one toggle clamp and other attaching means to attach other parts of the surgical retractor firame system.

For illustration purposes, Figures 1A through 1C depict an embodiment of the surgical retractor frame system that utilizes several toggle clamps. Figure 1A is a perspective view of surgical retractor frame system 1A. Table post 2A with an integral table post clamp 2B anchors surgical retractor frame system 1A to rail 20 on an operating table. Operating tables are typically outfitted by the manufacturer with rails 20, usually on at least both sides of the surgical table. Surgical retractor frame system 1A is securely anchored to provide a stable structure for holding hardware onto which retractor 6 or other equipment may be attached. It is well known in the surgical arts that there are many types of retractors. Retractor clamp 7 in Figure 1A is one embodiment of a clamp utilized to a retractor 6.

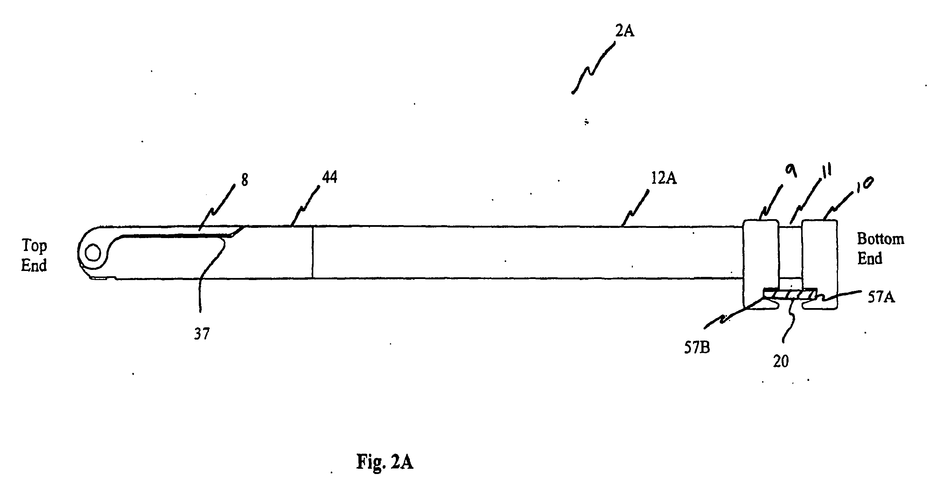

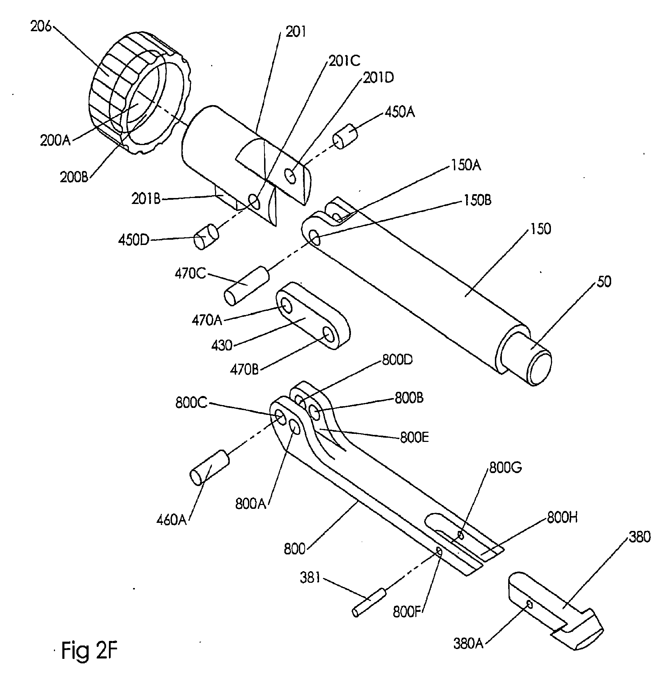

Referring to Figures 2A-2D, table post 2A has both a fixed clamp jaw 9 and a moving clamp jaw 10 at its lower end. Table post lever 8 is shown in a closed position. Lever 8 actuates moving jaw 10 through linkages (contained within table post cylinder 12A) between table post lever 8 and moving jaw 10. When table post lever 8 is moved from an open position to a closed position (as shown in Figure 1A), it draws moving jaw 10 upward towards fixed jaw 9 until jaws 10 and 9 are fully closed onto rail 20 and firmly clamped down upon both the top and bottom edges of rail 20 in a vise-like manner.

To attach the frame 1B to the table post 2A, the table post clamp block 27 of coupling clamp 3 (Figure 1A) is slid over table post 2A (with table post lever 8 in the closed position). Next, one end of extension beam 4 is slid into extension beam clamp block 26. After the user has oriented extension beam clamp block 26 and table post clamp block 27 to the desired positions and inserted extension beam 4 in extension beam clamp block 26 to achieve the desired working length of extension beam 4, coupling clamp lever 28 is moved into a closed position as shown in Figure 1A. The free end of extension beam 4 is connected to frameset clamp 13.

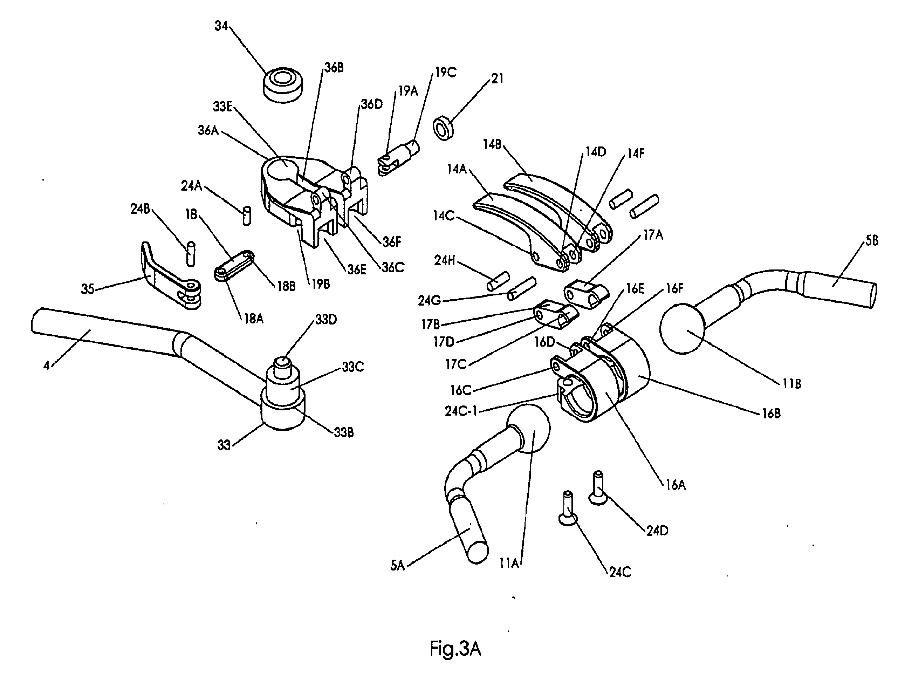

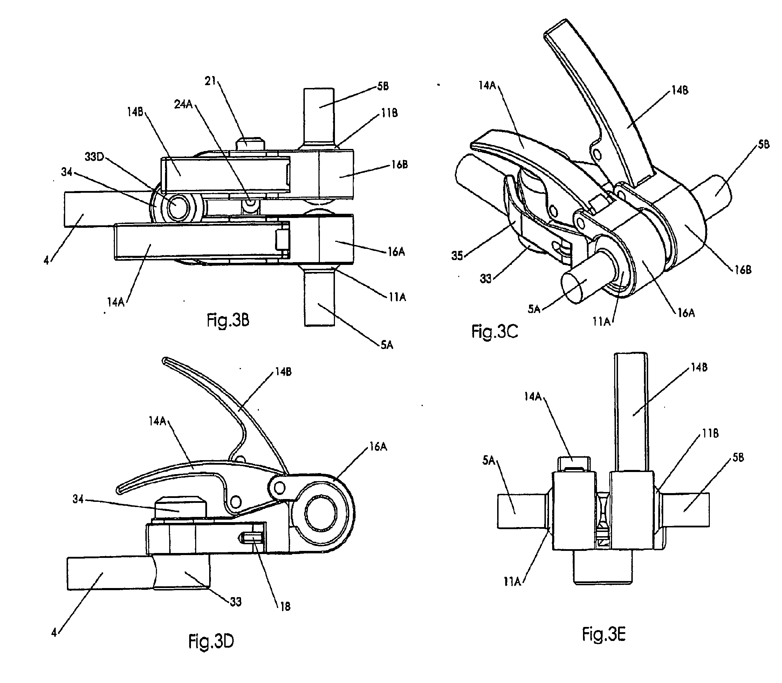

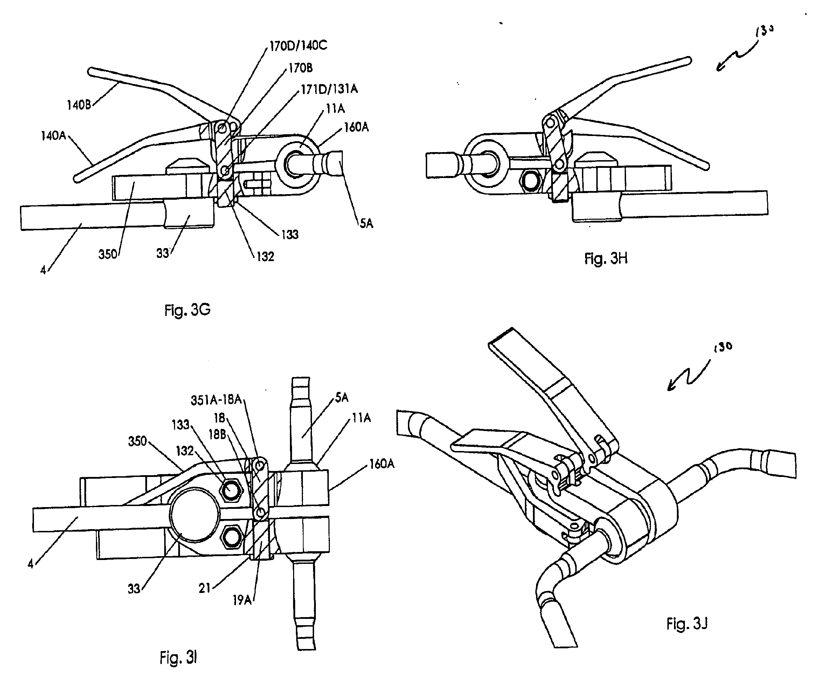

As shown in Figures 3A-3E, ball 11A at the ball end of support arm 5A is inserted into ball receiver 16A. Support arm lever 14A is then moved into a closed position, as shown in Figure 1A, thereby securely clamping ball 11A of support arm 5A into ball receiver 16A. While receivers 16A and 16B are disclosed as being ball receivers, they may also receive other shaped members, such as cylindrical or polygon shaped members. Retractor clamp 7A is clamped onto support arm 5A with retractor clamp lever 32.

As shown in Figures 1A and 3A, in one embodiment there are two support arms 5A and 5B. The embodiment of Figure 1A, however, may be assembled by the operator with one or more such support arms as needed. Moreover, one or more frames 1B may be attached to table post 2A by another coupling clamp 3. Furthermore, one or more retractor clamps may be clamped onto the same support arm or onto other support arms. In the embodiment illustrated in Figures 1A and 3A, ball receiver 16B, support arm 5B and retractor clamp 7B can be connected to each other in the same manner that support arm ball receiver 16A, support arm 5A and retractor clamp 7A are connected to each other. Retractor clamps 7A and 7B may be used for clamping surgical equipment, such as a retractor onto the surgical retractor frame system 1A to hold open an incision during surgery. Although there are many kinds of retractors, for illustration purposes, a typical retractor, retractor 6, is shown clamped to retractor clamp 7B.

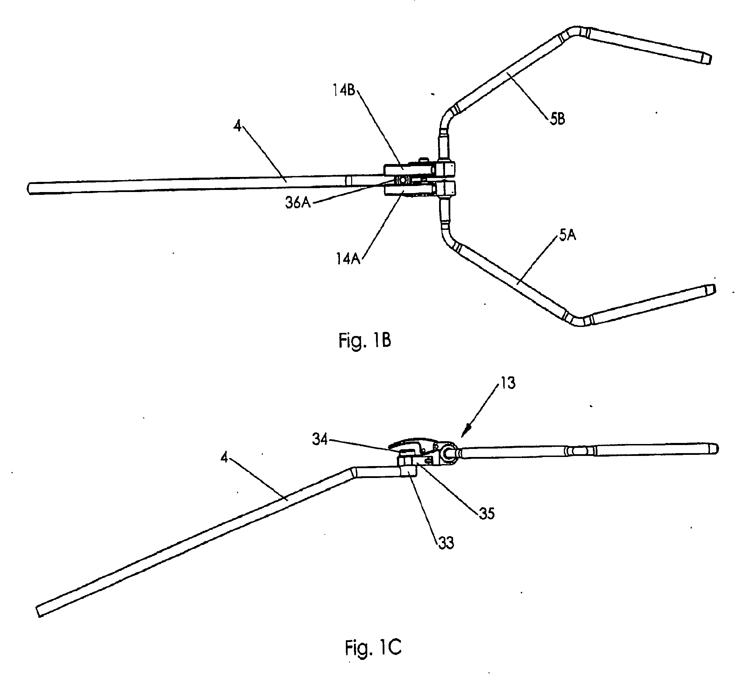

Figure 1B is a top view and Figure 1C is a side view of an embodiment of frame 1B (retractor clamp not shown). Figures 1C and 3A reveal a beam swivel 33 on the end of beam extension 4 proximal to frameset clamp 13. Beam swivel 33 projects through frame body 36A and is movably connected to frame body 36A by beam head 34. Figures 1A, 1B, and 1C point out swivel lock lever 35 (shown in Figure 1A below frameset lever 14A), which locks beam swivel 33 in place through the use of a toggle clamp mechanism, after it is oriented by operator at the desired position.

There are various combinations of clamping elements that can provide the toggle clamping table post of the present invention. For illustration purposes, Figures 2A through 2D depict one such combination.

Figure 2A is an elevation view of an embodiment of the table post of the present invention. In Figure 2A, table post 2A is shown with lever 8 in a closed position at the top end of table post 2A. Fixed and moving jaws 9 and 10 are at the bottom end. When lever 8 is in a closed position as shown in Figure 2A, it is stowed in lever stowage 37 in linkage body 44 so the outside wall of lever 8 is flush with the outside wall of linkage body 44. The outside diameter of linkage body 44 and of cylinder 12A is the same. Keeping the diameter of linkage body 44 and cylinder 12A of table post 2A the same along their entire length allows the inside diameter of table post clamp block 27 to fit over linkage body 44 and be moved along linkage body 44 and/or cylinder 12A to a point where the operator wishes to clamp coupling clamp 3. It conversely allows coupling clamp 3 to be slid up and over top of table post 2A for removal. The inside diameter of table post clamp block 27 is designed to be slightly larger than the outside diameters of linkage body 44, closed lever 8, and cylinder 12A and yet close enough that when coupling clamp 3 is clamped, it is securely affixed to table post 4.

When lever 8 is in a closed position, as shown in Figure 2A, moving jaw 10 is positioned in close proximity to fixed jaw 9 to the degree that it firmly grips table rail 20. Guide rod 11 is fixedly attached to, or integral with, moving jaw 10 and moves axially within cylinder 12A. The diameter of guide rod 11 is smaller than the inside diameter of cylinder 12A so that guide rod 11 is able to move axially within cylinder 12A.

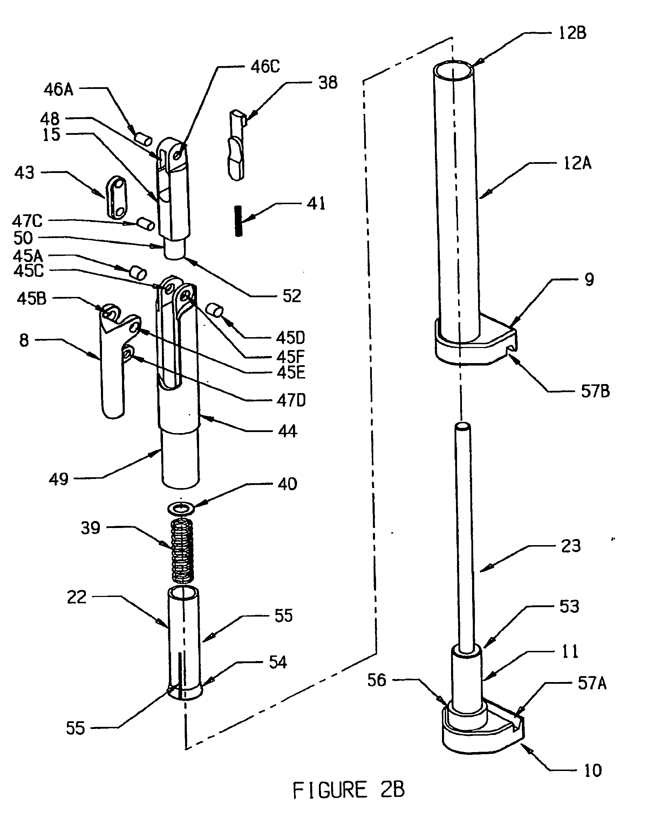

Figure 2B is an exploded view of table rail post 2A with integral table post clamp 2B. Connecting rod 23 is fixedly attached to, or integral with, guide rod 11 and guide rod 11 is fixedly attached to, or integral with, moving jaw stop 56. Moving jaw 10 is also comprised of rail capture 57A and is in-line with rail capture 57B of fixed jaw 9. When lever 8 of table post 2A is in the closed position, table rail 20 is clamped between rail captures 57A and 57B as shown in Figure 2A.

As shown in Figure 2B, a portion of linkage body 44 is received in cylinder bore 12B and secured by any of various securing means known in the art. For example, in one embodiment, reduced section 49 of linkage body 44 has an outside diameter that is about the same or slightly larger than the inside diameter of cylinder 12A so that they will be held together by an interference fit. Lever 8 is hinged to linkage body 44 by link pins 45A and 45D. Link pin 45A extends through lever aperture 45B of lever 8 and aperture 45C of linkage body 44. Link pin 45D extends through aperture 45E of lever 8 and into aperture 45F of linkage body 44. An intermediate link 43 is hinged in clevis 48 by link pin 46A inserted through a first aperture 46C of slide link 15, 47A of intermediate link 43, and a second aperture (not shown) of slide link 15 located opposite of and coaxial with first aperture 46C. Slide link 15 is indirectly hinged to lever 8 by link pin 47C. Link pin 47C is inserted through lever aperture 47D and intermediate link aperture 47B.

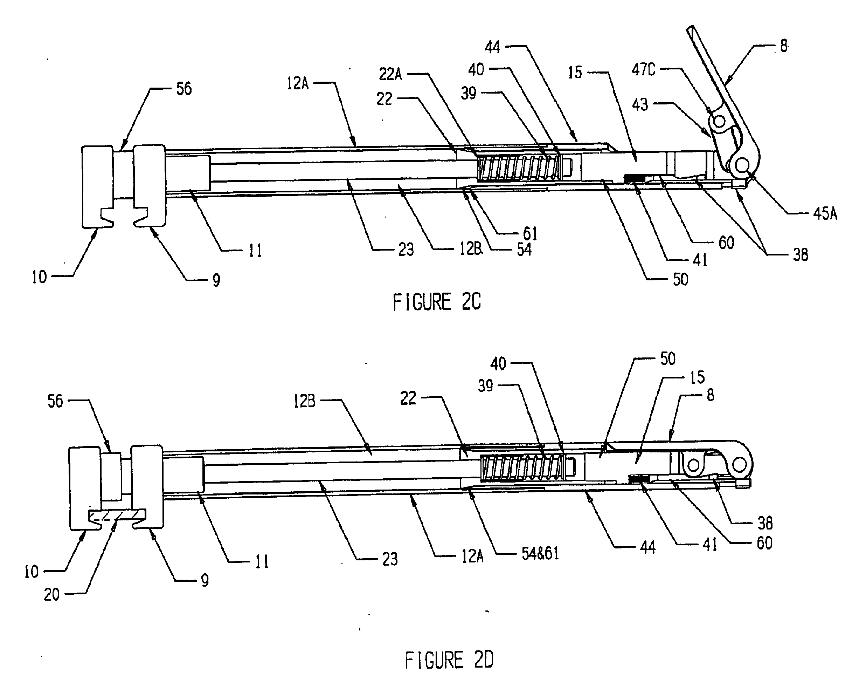

Figures 2C and 2D are cut-away views of table post 2A in an unclamped position and clamped position, respectively. Reduced section 50 of slide link 15 is threaded for mating engagement with inside threaded collet 22. In Figure 2C when lever 8 is in the raised/unclamped position, the slide link 15 and collet 22, which are threaded together, are lowered in cylinder 12A. In this position mating taper 61 of linkage body 44 ceases to compress tapered end 54 of collect 22. A comparison between Figures 2C (lever 8 open) and 2D (lever 8 closed) shows that when lever 8 is closed, tapered end 54 of collet 22 is pressed against the inside of mating taper 61 of linkage body 44, compressing longitudinal slit 55 of collet 22. Compression of longitudinal slit 55 reduces the diameter of the inside bore of collet 22 to a smaller size, thus compressing collet 22 onto connecting rod 23. Consequently, compression of the outside surface of collet 22 in the foregoing manner results in compression upon connecting rod 23 which thereby locks it in position until lever 8 is opened. In Figure 2D connecting rod 23 is in a position that securely clamps table rail 20 between fixed jaw 9 and moving jaw 10. On the other hand, jaws 9 and 10 of Figure 2C are open. As lever 8 is opened to the position shown in Figure 2C, slide link 15 presses collet 22 downward, driving tapered end 54 out of mating taper 61, thereby releasing the tightening action of collet 22 on connecting rod 23.

In one embodiment of the surgical retractor frame system 1A, moving jaw 10 is drawn towards fixed jaw 9 when lever 8 is open and table post 2A is unclamped. Thus, the operator does not have to hold the bottom of moving jaw 10 up against the bottom of rail 20 while closing lever 8. This eases operator use and allows operator to attach table post 2A to rail 20 while keeping his or her hands inside the sterile field of the surgery.

There are various means of drawing moving jaw 10 toward fixed jaw 9 when lever 8 is open. One embodiment of such means is illustrated herein Figures 2B, 2C and 2D, and includes the cooperation of a retaining ring 40, main spring 39 and upper surface 22A of collet 22. The diameter of main spring 39 is sized so that it is able to move within inside diameter of collet 22 without binding. Retaining ring 40 is attached to connecting rod 23 near the top of rod 23 thereby retaining the top of main spring 39 near the top of connecting rod 23. Upper surface 22A retains the bottom of main spring 39. Main spring 39 is assembled within collet 22 so that it is in a compressed state whether lever 8 is open or closed. In this manner main spring 39 presses against retaining ring 40 thereby pulling or biasing connecting rod 23 upward to pull moving jaw 10 toward fixed jaw 9. When table post 2A is affixed to table rail 20 while lever 8 is open, table rail 20 is squeezed but not locked between moving jaw 10 and fixed jaw 9 by the force of main spring 39. Closing lever 8 draws collet 22 into mating taper 61, thereby squeezing collet 22 onto connecting rod 23, whereby connecting rod 23 and attached moving jaw 10 are locked in position relative to fixed jaw 9.

To secure table post 2A to an operating table for the embodiment described above, the operator grasps table post 2A near the top end. The operator then rotates the bottom end of table post 2A upward at an angle toward table rail 20 such that moving jaw 10 can be hooked onto the bottom of rail 20 without engaging fixed jaw 9. The operator then lifts the top end of table post 20 thereby moving fixed jaw 9 away from moving jaw 10 sufficiently so that the operator can bring table post 2A to a vertical position with fixed jaw 9 over the top of table rail 20. The operator then stops lifting the top end of table post 20, allowing fixed jaw 9 to engage the top of rail 20, whereby rail 20 is squeezed between fixed jaw 9 and moving jaw 10. If necessary, the operator is now free to adjust the lateral position of table post 2A by sliding it along rail 20. When the desired position is achieved, the operator closes lever 8 in order to fixedly clamp table post 2A to rail 20.

Figures 2B, 2C and 2D illustrate release slide 38 and return spring 41. Lock lever 8 snaps or clicks into the locked position when the pivot point 47B attachment of the lock lever 8 and intermediate link 43 passes "over center" through the line defined by points 46A and 47A. This over center condition keeps the mechanism in the lock position and is sufficiently tight that it may be difficult for an operator to simply lift the lock lever 8 to release the mechanism. Thus, in one embodiment a release slide 38 is provided. Release slide 38 is a sliding wedge with a projection (button) which passes out the end (top) of the post assembly. The operator may release the lock mechanism by pressing on the button and thus sliding the wedge under the pivot point 27B and driving the pivot point back through the over center line. In order that the sliding wedge of the release slide 38 does not stay down in the release position making it impossible to lock the device, a spring 41 is captured in the slide pocket. Return spring 41 maintains an upward spring force on release slide 38 that retains release slide 38 in the release slide space 60. Release slide space 60 is a cavity that retains release slide 38 and return spring 41.

The threaded connection of the slide link 15 and the collet 22 both attaches the collet to the drive mechanism and provides for adjustment of how far the collet 22 draws into the mating taper of the linkage body 44 thus allowing for adjusting how tightly the collet 22 clamps onto the connecting rod 23. This adjustment is generally made during assembly and set-up of the device and not typically by the end user.

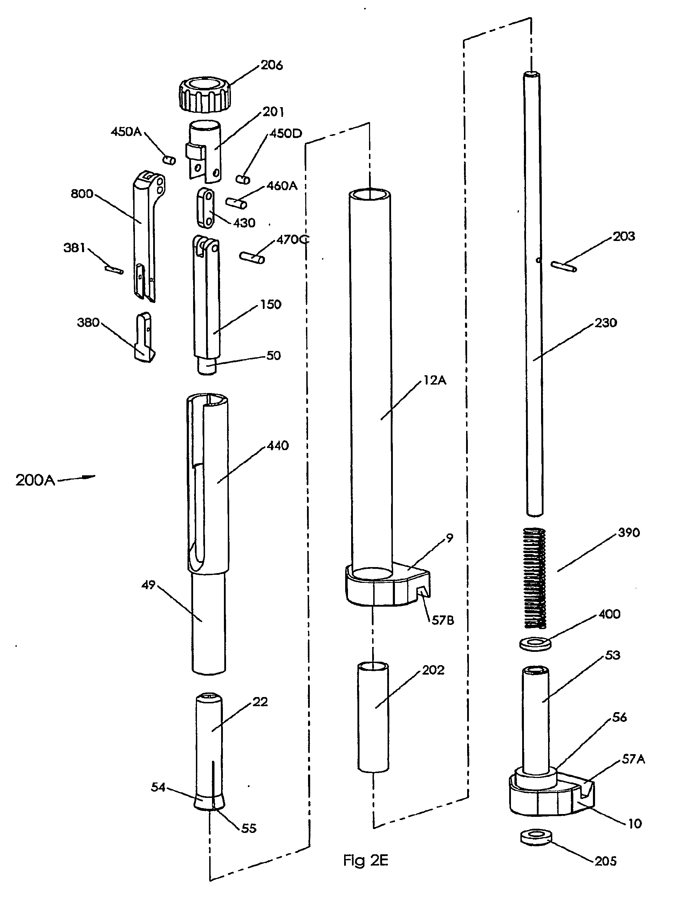

In another embodiment of the surgical retractor frame system, an alternative table post clamp 2B for adjusting how far collet 22 draws into the mating taper is illustrated in Figures 2E through 2H. In this embodiment, adjustor 206 is threaded onto lever mount shuttle 201. Adjuster 206 may be threaded onto shuttle 201 with a female threaded portion 200A in cooperation with male threaded portion of shuttle 201, or a threaded bolt may extend from adjuster 206 into a female threaded receptacle disposed within shuttle 201. Further, adjuster 206 may be hidden from operator view or exposed as shown in Figures 2E through 2H.

Shuttle 201 is attached to lever 800 by means of pin 450D inserted through apertures 201C and 800A, and by means of pin 450A inserted through aperture 201D and 800D. Lever 800 is attached to intermediate link 430 by means of pin 460A inserted through apertures 800C and 800D of lever 800 and aperture 470A or intermediate link 470A. In an alternative embodiment of the present invention, lever 800 may have only one aperture through which pin 460A inserts to attach lever 800 to intermediate link 430 at aperture 470A.

Intermediate link 430 is attached to slide link 150 with pin 470C which is inserted into apertures 150A and 150B of slide link 150 and through aperture 470B of intermediate link 430.

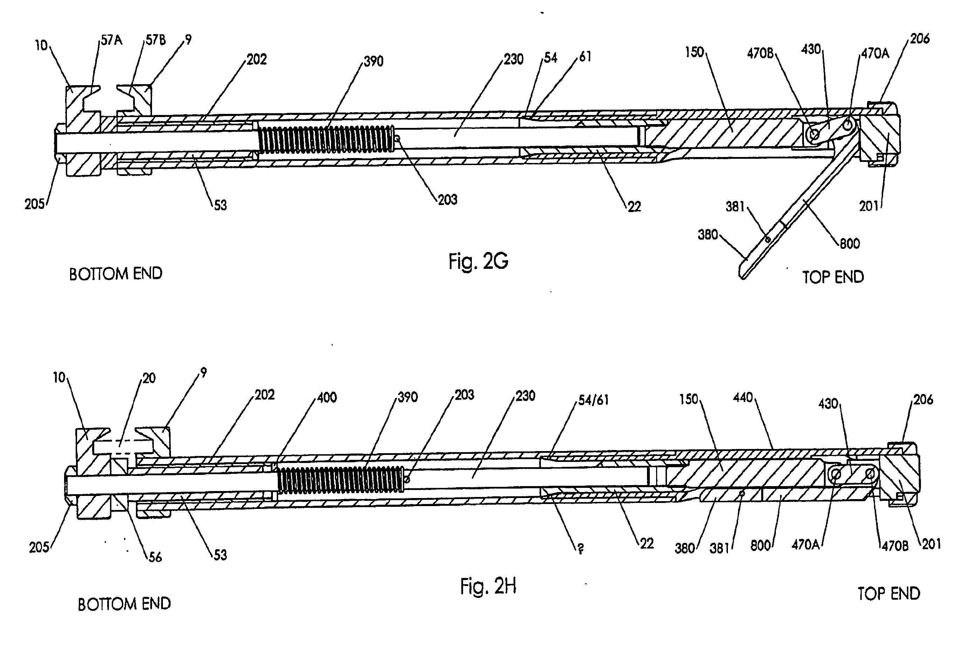

Figures 2G and 2H are cut-away views of table post 200A in an unclamped position and clamped position, respectively. Reduced section 50 of slide link 150 is threaded for mating engagement with inside threaded collet 22, however, it is also know that they may be attached by any convenient means. In Figure 2G, when lever 800 is in the raised/unclamped position, threaded together slide link 150 and collet 22 are lowered in cylinder 12A. In this position mating taper 61 of linkage body 440 ceases to compress tapered end 54 of collect 22. A comparison between Figures 2G (lever 8 open) and 2H (lever 8 closed) shows that when lever 8 is closed, tapered end 54 of collet 22 is pressed against the inside of mating taper 61 of linkage body 440, compressing longitudinal slit 55 of collet 22. Compression of longitudinal slit 55 reduces the diameter of the inside bore of collet 22, thus compressing collet 22 onto connecting rod 230. Consequently, compression of the outside surface of collet 22 in the foregoing manner results in compression upon connecting rod 230 which thereby locks it in position until lever 800 is opened.

In Figure 2H connecting rod 230 is in a position that securely clamps table rail 20 between fixed jaw 9 and moving jaw 10. Conversely, in Figure 2G, jaws 9 and 10 are open. As lever 8 is opened to the position shown in Figure 2G, slide link 150 presses collet 22 downward, driving tapered end 54 out of mating taper 61, thereby releasing the tightening action of collet 22 on connecting rod 230.

In a preferred embodiment, moving jaw 10 is drawn toward fixed jaw 9 by means illustrated in Figures 2E through 2H. Figures 2E through 2H depict the cooperation of a spring shelf washer 400, main spring 390 and spring stop pin 203 to provide a temporary spring clamp such that jaws 9 and 10 are drawn or biased toward each other even when lever 800 is open. The lower part of connecting rod 230 is placed through the inside diameter of spring 390 until the upper part of spring 390 contacts spring stop pin 203. The length of pin 203 is longer than the diameter of spring 390, whereby spring 390 is retained below pin 203. The lower part of spring 390 rests against and is retained by spring shelf washer 400.

The inside diameter of washer 400 is smaller than the inside diameter of bushing 202 while its outside diameter is substantially the same as the outside diameter of bushing 202. Washer 400 rests against and is retained by the top surface of bushing 202. Alternatively, washer 400 may be integral with the top of bushing 202.

Bushing 202 is longer along its axial dimension than guide rod 53. The inside diameter of bushing 202 is larger than the outside diameter of guide rod 53 such that rod 53 can be inserted into bushing 202 and travel freely along its axis. Bushing 202 is permanently inserted into and is thus integral with the lower part of table post cylinder 12A such that the lower end of bushing 202 is substantially concentric with the lower end of cylinder 12A. Bushing 202 and cylinder 12A are attached by any means known in the art such as press fitting or pinning. In a highly preferred embodiment, bushing 202 is made of a substantially friction reducing material with a high enough melting temperature such that it can be autoclaved without being substantially degraded, such as a polyketone plastic, thus enhancing the ability of connecting rod 230 to travel through table post cylinder 12A while still allowing for sterile surgical procedure. The diameter of main spring 390 is sized so that it is able to move within inside diameter of collet 22 and along the outside diameter of connecting rod 230 without binding.

Connecting rod 230 is attached to guide rod 53. Spring stop pin 203 is inserted into an aperture in connecting rod 230 at a suitable location such that main spring 390 is in a compressed state whether lever 800 is open or closed. In this manner main spring 390 presses against stop pin 203 thereby pulling connecting rod 230 upward, whereby moving jaw 10 is pulled toward fixed jaw 9. When table post 200A is affixed to table rail 20 while lever 800 is open, table rail 20 is squeezed but not locked between moving jaw 10 and fixed jaw 9 by the force of main spring 390. Closing lever 800 draws collet 22 into mating taper 61, thereby squeezing collet 22 onto connecting rod 230, whereby connecting rod 230 and attached moving jaw 10 are locked in position relative to fixed jaw 9.

A preferred embodiment of the present invention, utilizes a release lever 380, as illustrated in Figures 2E through 2H. Release lever 380 attaches to table post lever 800 by release lever pin 381. Lever 800 is hinged to lever mount shuttle by link pins 450A and 450D. Link pin 450A extends through lever aperture 800B of lever 800 and aperture 201D of lever mount shuttle 201. Link pin 450D extends through aperture 800A of lever 800 and into aperture 201C of lever mount shuttle 201. An intermediate link 430 is hinged in clevis 800E by link pin 460A inserted through a first aperture 800D of lever 800, aperture 470A of intermediate link 430, and aperture 800C of lever 800. Slide link 150 is hinged to intermediate link 430 by by link pin 470C. Link pin 470C extends through apertures 150A and 150B of slide link 150 and intermediate link aperture 470B. Slide link 150 is attached to collet 22 and causes collet 22 to clamp connecting rod 230 in the same manner as slide link 15, collet 22 and connecting rod 23 in figures 2A through 2D.

In a preferred embodiment, release lever 380, table post lever 800, linkage body 440 and slide link 150 are designed such that release lever 380 and table post lever 800 fit into a concavity in linkage body 440 such that a tubular profile is presented onto which coupling clamp 3 or other devices may be slid.

Lever 800 snaps or clicks into the lock position when the pivot point located approximately at pin 460 (pivot 2) passes "over center" through the line defined by a pivot located approximately at pins 450A and 450D (pivot 1) and a pivot point located . approximately at pin 470C (pivot 3). This over center condition keeps the mechanism in the lock position and is sufficiently tight that it may be difficult for an operator to simply lift the lock lever 800 to release the mechanism. Thus, release lever 380 is provided.

Release lever 380 is a small lever built into larger lever 800. The operator may easily begin to lift release lever 380 by sliding a thumb under its distal end causing lever 380 to rotate about pin 381. Said rotation causes the proximal end of release lever 380 to press against slide link 150, which is partly revealed by an opening in linkage body 440. As the proximal end of release lever 380 presses against slide link 150, lever 800 is forced open until it drives pivot point 2 over the over center line, thus releasing lever 800 from the lock position.

There are various combinations of elements that can provide the toggle clamping frameset clamp. For illustration purposes, Figures 3A through 3E describes one such combination. Figure 3A is an exploded view of an embodiment of one frameset clamp 13. In Figure 3A, frameset clamp 13 is the attachment point for support arms 5A and 5B on one end of frame 1B and extension beam 4 on the other end of frame 1B, as also shown in Figures 1B and 1C.

As shown in Figure 3A, extension beam 4 terminates at one end in beam swivel 33. Beam swivel 33 is comprised of stop 33B, axle 33C, and neck 33D. Axle 33C is inserted into axle receiver 33E until stop 33B abuts the bottom of frame body 36A. Neck 33D protrudes above frame body 36A and is affixed in place by beam head 34 to arrest vertical movement. However, axle receiver 33E is free to rotate about axle 33C until the operator orients support arm 5 to a desired location relative to table post 2A. The operator then closes swivel lock lever 35 by moving it towards frame body 36A. As swivel lock lever 35 closes, intermediate link 18 (attached to swivel lock lever 35 by pin 24B and to draw link 19A by pin 24A) draws draw link 19A ever tighter through draw link channel 19B. Draw link 19A is affixed at its head-end 19C by head 21 to arrest its movement out of draw-link channel 19B. When swivel lock lever 35 is fully closed, the linkage combination of swivel lock lever 35, intermediate link 18, and draw link 19A acting through draw-link channel 19B squeezes frame body gap 36B against swivel 36C to firmly secure it in place.

Ball 11A on one end of support arm 5A and ball 11B on one end of support arm 5B are inserted into ball receivers 16A and 16B respectively. Ball receivers 16A and 16B are also referred to as first and second clamp members, and axel receiver is also referred to as the second clamp member. Each of the clamp members, in a preferred embodiment, have an arcuate jaw surface, that being the interior surface of the clamp members. Axel receiver has a clamp axis transverse to the clamp axis of the first and second clamp members, and in one embodiment, is orientated approximately 90° to a plane of the clamp axis of the first and second clamp members. Ball receivers preferably have a portion of the interior thereof that is a concave shaped receiver to accept a spherical member. Ball receiver 16A is connected to frame body 36A by pin 24C. Pin 24C extends through pin aperture 24C-1 in ball receiver 16A and is fastened to the underside of projection channel 36E of frame body 36A. Ball receiver 16B is connected to frame body 36A in a similar manner, such that pin 24D extends through a similarly placed pin aperture in ball receiver 16B and is fastened to the underside of projection channel 36F of frame body 36A.

Lever 14A is connected to intermediate link 17B as follows. Pin 24H extends through aperture 14C of lever 14A, aperture 17D of intermediate link 17B and finally through an aperture in lever 14A that is opposite and coaxial with aperture 14C. Lever 14B is connected to intermediate link 17A in a similar manner.

Lever 14A and connected intermediate link 17B are connected to frame body 36A as follows. Lever 14A is placed over frame body 36A such that the bottom surface of curved finger 17C of intermediate link 17B mates with the top of hinge 36C. Pin 24G extends through aperture 14D of lever 14A, an aperture located in hinge 36C of frame body 36A and finally through aperture14F of lever 14A. Lever 14B and connected intermediate link 17A are connected to and mate with frame body 36A and hinge 36D of frame body 36A in a similar manner.

Although levers 14A and 14B are capable of operating either independently or simultaneously, for illustration purposes only they are described herein operating at the same time. When levers 14A and 14B are opened (in an up position) intermediate links 17A and 17B are raised and relieve clamping force on ball receivers 16A and 16B. When levers 14A and 14B are closed (in a down position) intermediate links 17A and 17B are also forced down pushing the top ends of ball receivers 16A and 16B down and squeezing the gaps in ball receivers 16A and 16B together against balls 11A and 11B to securely fix them in the desired position. For clarity, Figures 3B through 3E show various other views of frameset clamp 13.

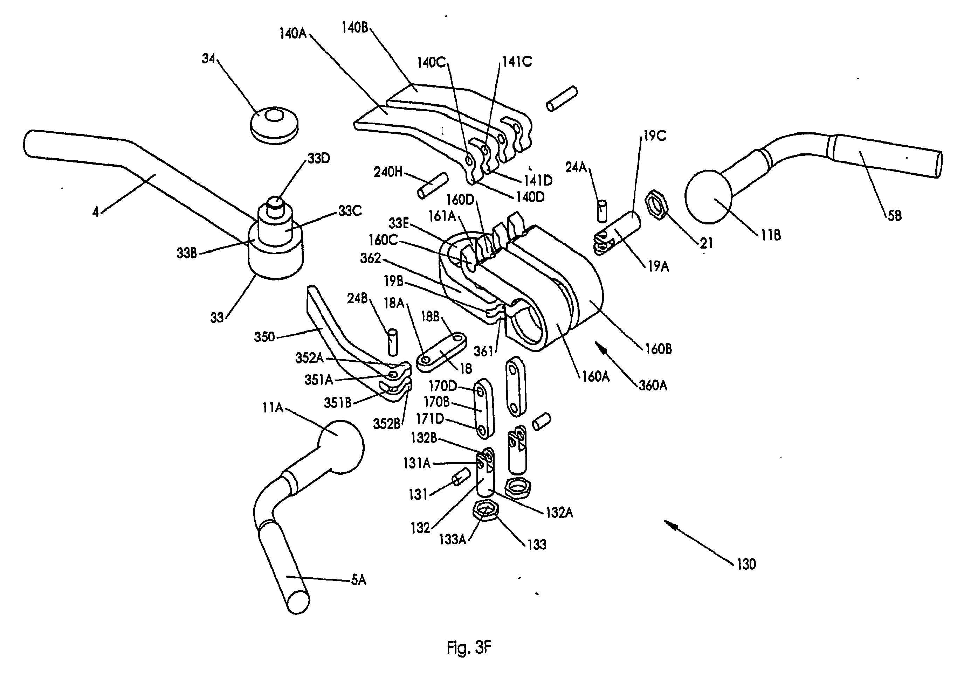

Figures 3F through 3H illustrate another embodiment having features that may be practiced alone or in concert with any of the other embodiments to provide a desired clamp. In one modification, illustrated in Figure 3F, a mechanism for adjusting clamp tension is provided. In one embodiment such mechanism includes an adjusting head 133 that is screwed onto draw bolt 132 with mating threads 133A and 132A on each, respectively. Draw bolt 132 is attached to intermediate link 170B by pin 131 which extends through apertures 132A and 132B of draw bolt 132 and 171D of intermediate link 170B.

Draw bolt 132 extends through an aperture (not shown) in the base 362 of frame body 360A. The aperture in the base 362 of the frame body 360A is directly in line with clevis 161A. Intermediate link 170B extends through clevis 161A in frame body 360A. Intermediate link 170B is attached to lever 140A by pin 240H which extends through aperture 170D of intermediate link 170B and through apertures 140C and 141C of lever 140A.

When adjusting head 133 is tightened, it compresses the distance between base 362 of frame body 360A and the distal end of ball receiver 160A. Thus when lever 140A is moved from the open to the closed position, the circumference of the inside, clamping surface of ball receiver 160A is correspondingly tighter than it would have been had adjusting head 133 not been tightened, or been tightened to a lesser amount.

In another embodiment, ball receiver 160B is adjustable by its own separate adjusting head, draw bolt, intermediate link and lever 140B all cooperating and connected in substantially the same manner as was described for ball receiver 160A.

In another embodiment, axle receiver 33E is adjustable in substantially the same manner as was described for ball receiver 160A by adjusting head 21. Adjusting head 21 is threaded onto draw link 19A which is connected to intermediate link by pin 24A which extends through an aperture or apertures in draw link 19A and aperture 18B in intermediate link 18. Intermediate link 18 is connected to lever 350 by pin 24B which extends through aperture 18A of intermediate link 18 and apertures 351A and 351B of lever 350. After tightening or loosening head 21, swivel lock lever 350 is fully closed, whereby the linkage combination of swivel lock lever 350, intermediate link 18, and draw link 19A acting through draw-link channel 19B squeezes frame body gap 36B against axle 33C to firmly secure it in place. Typically, such adjustments in clamp tension using an adjusting means other than a lever are performed by the manufacturer of said clamps rather than by the end user. Preferably, such adjusting means comprising adjusting heads 133A and 21 as used in frameset clamp 13 or 130, or adjustor 206 as used in table post 2A or 200A are hidden from view after manufacture and during the use of said clamps by an end user.

Figures 3F through 3H illustrate a preferred embodiment (the 3F clamp) of the present invention which has fewer parts than the embodiments illustrated in Figures 3A through 3E (the 3A clamp). For example, with respect to frame 3A, the frame body 36A and ball receivers 16A and 16B are separate blocks which needed to be connected together with pins 25C and 24D respectively. Conversely, in the 3F clamp corresponding frame body 360A is one piece. In another example, the 3A clamp has pins 24C, 24D, 24G and a pin corresponding to 24G associated with lever 14B, as shown in Figure 3A. In the 3F clamp, however, these pins are not required.

Referring to the clamp illustrated in Figure 3A, pins 24C, 24D, 24G and pin corresponding to 24G function as the first pivot point as explained herein. In the clamp illustrated in Figure 3F, however, a pin is not required to perform the function of the first pivot. Instead, for lever 140A and ball receiver 160A, the function of the first pivot is performed by the outer edges of lobes 140D and 141 D of lever 140A at the point they contact and slide along pockets 160C and 160D respectively. Accordingly, in embodiments where lever 140B and clamp 160B are present, their corresponding first pivot function is performed by lobes and pockets in corresponding locations.

Referring to the swivel clamping portions of frameset clamps illustrated in Figures 3A and 3F, the outer edges of levers 35 and 350, respectively, both function as a first pivot. However, in the 3F clamp, the outer edges, i.e. lobes 352A and 352B of lever 350 are elongated into lobe shapes, and receiving pocket 261 in frame body 360A is shaped to match, thus providing a capturing function for lobes 352A and 352B. Optionally, levers 140A and/or 140B also have lobes 140D and 141D, and the respective arms extending from the distal ends of ball receivers 160A and 160B optionally provide matching pocket shapes to receive and partially capture the lobes. This capturing function helps keep the levers in place and functional, and improves alignment of the three pivot points for an improved toggle clamping function.

A mechanical advantage may also be provided in clamp 3F by increasing the distance of pivot one from pivot two which is axially centered at the pins by which the three levers 140A, 140B and 350 are attached. This can result in improved output travel allowing for improved clamping.

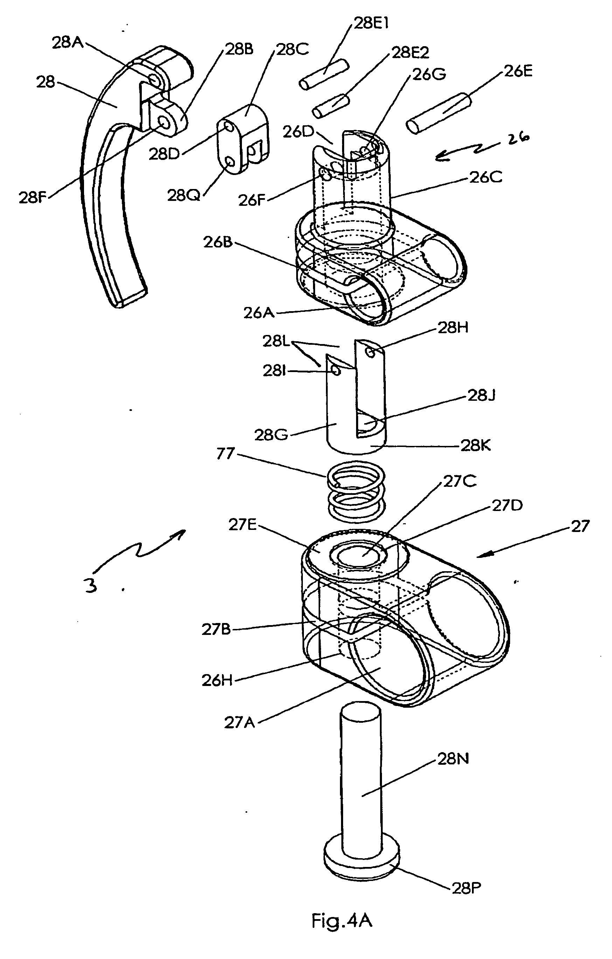

As explained herein, there are various combinations of elements that can provide the toggle clamping coupling clamp. For illustration purposes, Figures 4A through 4E depict one such combination as a representative embodiment.

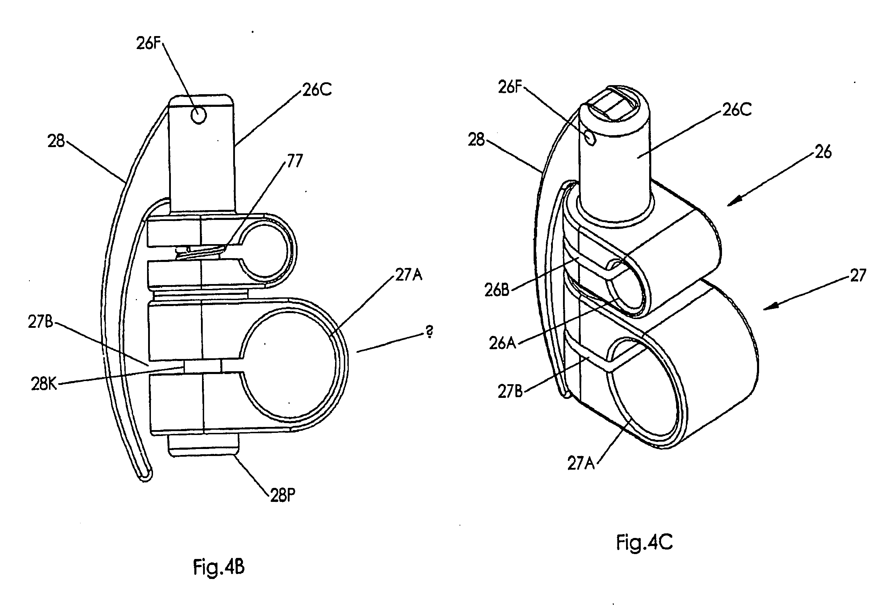

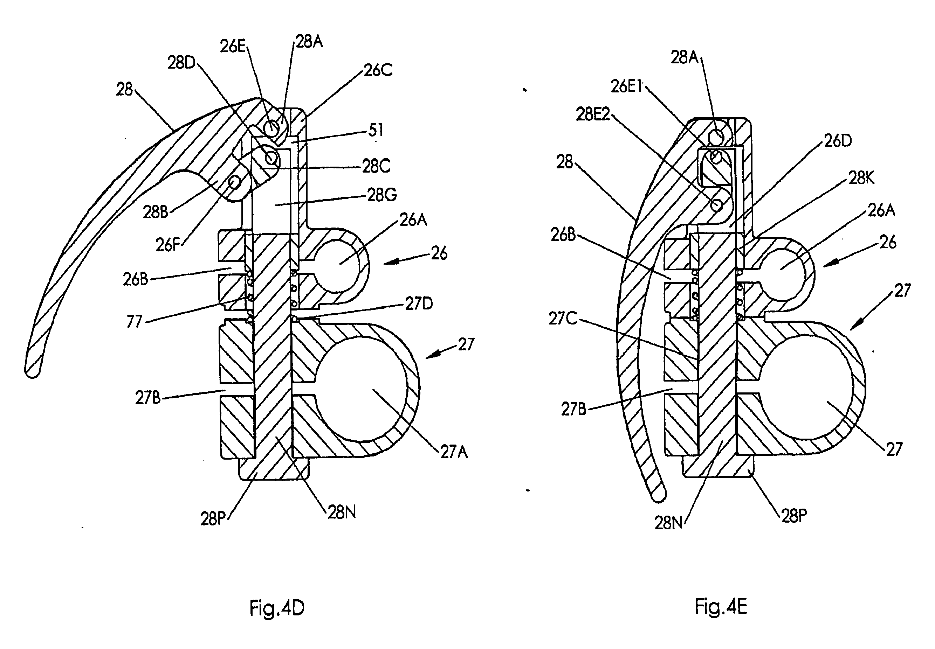

Figure 4A is an exploded view of coupling clamp 3. Figure 4B is a side view of coupling clamp 3. Figure 4C is a perspective view of coupling clamp 3. Figures 4D is a cut-away view of clamp 3 along a vertical axis, in an open position. Figure 4E is a cut-away view of coupling clamp 3 along a vertical axis in a closed position. Although the typical operating orientation of clamp 3 is depicted in Figure 1A, for illustration purposes in the description of clamp 3, directions such as "up," "upward," "upper" and "top of" refer to the direction of the tops of the sheets on which Figures 4D and 4E appear, whereas "down," "downward," "lower" and "bottom of" refer to the opposite direction.

The clamping elements of the coupling clamp 3 depicted in Figures 4A through 4E are extension beam clamp block 26 and table post clamp block 27. When lever 28 is closed, block 26 and block 27 simultaneously clamp. When lever 28 is opened, block 26 and 27 simultaneously unclamp.

When coupling clamp 3 is in the unclamped position, an operator slides table post aperture 27A over table post 2A and extension beam 4 through extension beam aperture 26A. After coupling clamp 3, table post 2A and extension beam 4 are in the desired position relative to one another, lever 28 is closed, thus clamping table post 2A to extension beam 4.

When lever 28 is closed, the slot 27B of table post clamp block 27 is squeezed closed by the combination of draw bolt 28N, slide link 28G, intermediate link 28C. Simultaneously, the slot 26B of extension beam clamp block 26 is squeezed closed by the combination of draw bolt 28N, slide link 28G, intermediate link 28C.

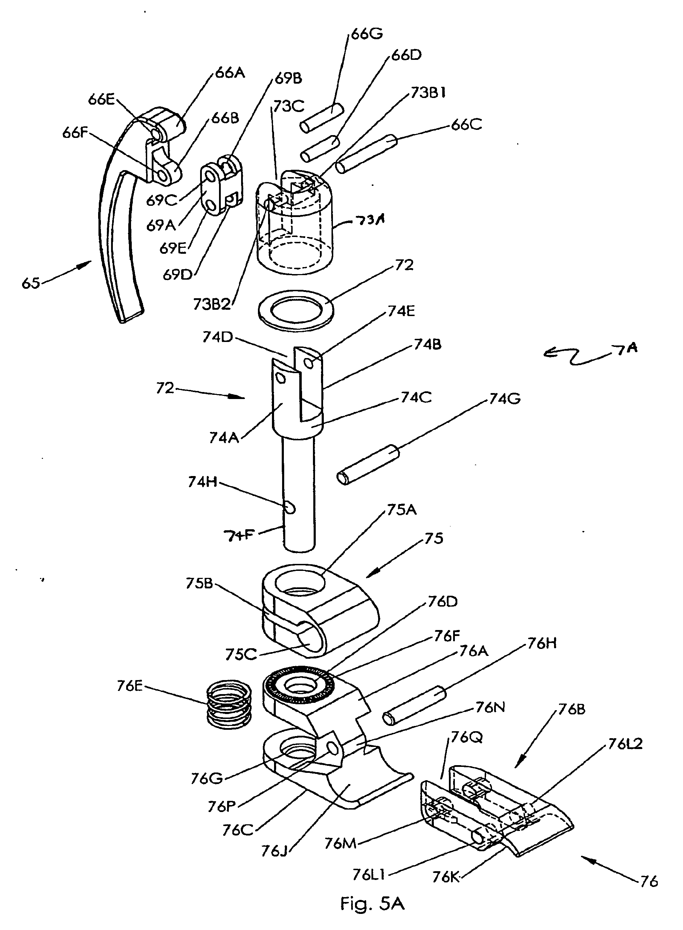

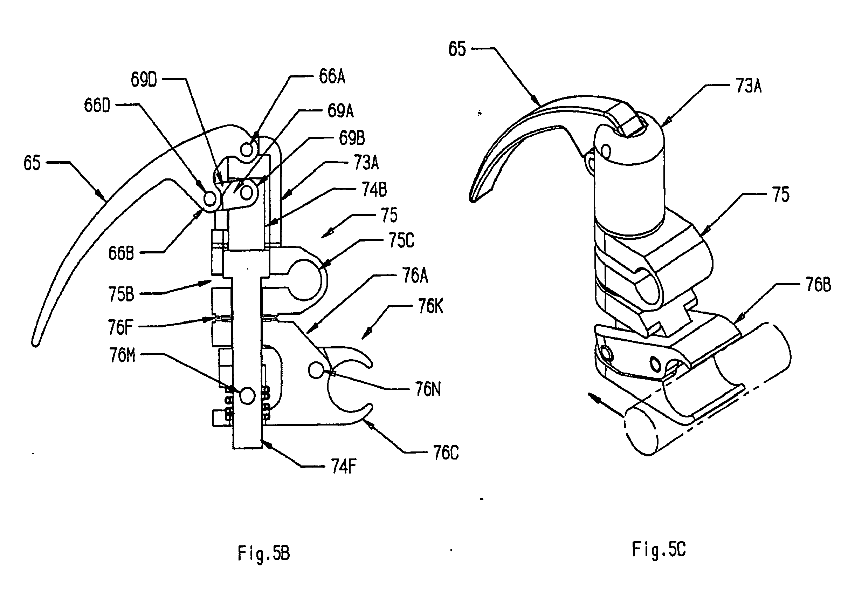

Further, in a preferred embodiment, in the closed position clamp blocks 26 and 27 are squeezed together, inhibiting rotation of clamp block 26 with respect to clamp block 27. Optionally, in an alternate embodiment mating radial locking serrations on the lower portion of clamp block 26 and the upper portion of clamp block 27 are provided. In the closed position, when clamp blocks 26 and 27 are squeezed together, said serrations contact and lock against each other to prevent clamp block 26 from rotating relative to clamp block 27. (For reference, the serrations in clamp blocks 26 and 27 of coupling clamp 3 have analogous positions and function to serrations 75F and 76F in clamp blocks 75 and 76 of retractor clamp 7, depicted in Figures 5A and 5B and further described herein.)

When lever 28 is opened, blocks 26 and 27 unclamp and no longer press against each other. Blocks 26 and 27 unclamp because coupling slots 26B and 27B respectively, are free of a squeezing force exerted by the clamping mechanism of coupling clamp 3. In addition, because they are free of said squeezing force, blocks 26 and 27 no longer press against each other and are thus free to rotate with respect to one another.

Optionally in an alternate embodiment, spring 77 is provided to assist coupling blocks 26 and 27 to disengage from one another when the lever 28 is opened. The circumference of spring 77 is such that spring 77 fits around draw bolt 28N but cannot fit into lower draw bolt aperture 27C of block 27 nor into hole 28J of slide link 28G. The lower end of spring 77 presses against spring retaining surface 27D of block 27 while the upper end of spring 77 presses against the underside of slide link 28G. Spring 77 is more compressed when lever 8 is in the closed position and less compressed when lever 8 is open.

The clamping mechanism of coupling clamp 3, comprising draw bolt 28N, slide link 28G, intermediate link 28C, and lever 28, clamps both table post clamp block 27 and extension beam clamp block 26 simultaneously when lever 28 is moved from the open position shown in Figure 4D to the closed position shown in Figure 4E.

As shown in Figure 4A, draw bolt 28N inserts from the bottom of coupling clamp 3 into and through lower draw bolt aperture 27C and spring 77. In various embodiments, the top of draw bolt 28N is fastened to slide link 28G through-hole 28J by mating threads, welding, pinning, or any other suitable means. In other embodiments of the present invention, draw bolt 28N and slide link 28G are manufactured as a single piece, and spring 77 is not provided, or it is positioned by other means or it is placed in a different position. Alternatively, bolt head 28P is separately manufactured and attached after spring 77 is placed around draw bolt 28N.

Two parallel prongs 28L of slide link 28G extend upwardly into upper draw bolt aperture 26H (Figure 4D) and into housing 26D of stack 26C. Upper draw bolt aperture 26H and lower draw bolt aperture 27C are in-line with one another as is housing 26D. Intermediate link 28C is attached to second lever hinge point 28B on lever 28 by second link pin 28E2 which extends through aperture 28Q of intermediate link 28C, aperture 28F of second lever hinge point 28B, and, finally, through an aperture on intermediate link 28C opposite of and coaxial with aperture 28Q. Intermediate link 28C is also attached to slide link 28G. It is attached by link pin 28E1, which is first inserted through slide link aperture 281, then through aperture 28D in intermediate link 28C, and finally through slide link aperture 28H. Lever 28 is connected to stack 26C by lever link pin 26E, which extends through stack aperture 26G, first lever hinge point 28A, and then through stack aperture 26F.

Clamping and unclamping of coupling clamp 3 is achieved by multiple toggle joints. The first toggle is formed by first lever hinge point 28A of lever 28 and lever link pin 26E in stack 26C. The second toggle is formed by second lever hinge point 28B of lever 28 and intermediate link 28C, hinged at aperture 28F. The third toggle is formed by intermediate link 28C and slide link 28G hinged by first slide link pin 28E1 through slide link apertures 28H and 281 and intermediate link aperture 28D. This over-the-center toggle locking mechanism allows clamp 3 to lock closed and also provides strong clamping forces without excessive input. Additionally, clamping or unclamping can be achieved with relatively short lever motions.

When hinged lever 28 is moved from the open position shown in figure 4D to the closed to the closed position shown in Figure 4E, second lever hinge point 28B is pushed into housing 26D, forcing intermediate link 28C, attached draw bolt 28N and draw bolt head 28P upward toward first lever hinge point 28A of lever 28. This action squeezes clamp blocks 26 and 27 in between draw bolt head 28P and first lever hinge point 28A. Because of this squeezing action, the spaces in coupling slot 27B, and table post aperture 27A are reduced, whereby table post 2A may be clamped into aperture 27A. Furthermore, the spaces in coupling slot 26B and extension beam aperture 26A are also reduced, whereby extension beam 4 may be clamped in into aperture 26. In a preferred embodiment of the present invention, clamp blocks 26 and 27 are also squeezed into a tensioned state, creating a spring like pressure between both draw bolt head 28P and first lever hinge point 28A.

When hinged lever 28 is opened, draw bolt 28N is released, allowing it drop down, as illustrated by the space in gap 51 as shown in Figure 4D. Freeing draw bolt 28N releases pressure on clamp blocks 26 and 27, whereby the clamping forces of clamp blocks 26 and 27 are removed.

Another embodiment of the coupling clamp is assembled in the same manner as retractor clamp 7A as illustrated in Figures 5A through 5E and described below. In such an embodiment, either or both of clamp blocks 26 and 27 that are provided in clamp 3 are replaced by the moving jaw/fixed jaw system of support arm block 76 of retractor clamp 7A, and are manufactured of suitable size for one clamp block to clamp table post 2A while the other clamps extension beam 4. Such a coupling clamp is used in a similar manner to coupling clamp 3. For example, an operator may either widen the moving jaw sufficiently to clip the clamping portion over the table post cylinder 12A laterally, or slide the clamping portion onto table post cylinder 12A from the lever end.

There are various combinations of elements that can provide embodiments of the retractor clamp. For illustration purposes, Figure 1A and Figures 5A through 5E depict one such combination. Figure 5A is an exploded view of an embodiment of the retractor clamp of the present invention. In Figure 1A, retractor clamp 7A is fastened to a support arm 5A and it in turn clamps various types of surgical equipment such as a retractor in place. For example, Figure 1A depicts retractor 6 clamped to a retractor clamp 7B of the present invention in an orientation useful for holding open surgical wounds. For reference, Figure 1A also depicts a retractor clamp 7A of the present invention clamped to support arm 5A.

For illustration purposes in the description of clamp 7A, references to directions such as "raised" "up," "upward," "upper" and "top of" refer to the end of clamp 7A closest to the top of the sheet on which Figure 5B appears, whereas "lowered" "down," "downward," "lower" and "bottom of" refer to the end of clamp 7A closest to the bottom of the sheet on which Figure 5B appears.

As shown in Figure 5A, retractor clamp 7A uses a multi-link toggle mechanism. Clamp 7A simultaneously clamps (or unclamps) clamping portion 75C and the combination of moving jaw block 76B and fixed jaw block 76A. The clamp of clamping portion 75C is also referred to as the second clamp having a third arcuate jaw surface that is adapted to compress to a smaller perimeter upon movement ofthe output link to a second position. The jaw blocks form a clamp defined by a first clamp member having an first arcuate jaw surface and a second clamp member having a second arcuate jaw surface facing the first arcuate jaw surface. Thus clamping portion 75C can clamp retractor 6 while jaw blocks 76B and 76A clamp support arm 5A. Optionally, in an embodiment of the present invention, additional simultaneous clamping of fixed jaw block 76 to clamping portion 75 is provided to inhibit rotation of block 76 with respect to portion 75. Retractor clamp 7A also provides simultaneous unclamping of jaws 76B and 76C, clamping portion 75C and, optionally, unclamping of clamping of block 76 to portion 75.

In one embodiment the multi-link toggle mechanism allows lever 65 to unclamp retractor clamp block 75 and support arm block 76 with very little travel of lever 65. Lever 65 is inserted in housing 73C of draw bolt head 73A and hinged at first hinge point 66A by link pin 66C through coaxial apertures 73B1 and 73B2 of draw bolt head 73A, and through aperture 66E at first hinge point 66A. Second hinge point 66B of lever 65 is inserted into clevis end 69D of intermediate link 69A so both may pivot around link pin 66D inserted through coaxial apertures 69E of intermediate link 69A and aperture 66F of second hinge point 66B. Slide link portion 74A of draw bolt/slide link combination 72 inserts into draw bolt head 73A for slidable vertical movement therein. First end 69B of intermediate link 69A is inserted between parallel prongs 74B of slide link portion 74A and link pin 66G is inserted through coaxial apertures 74E of prongs 74B and coaxial apertures 69C of intermediate link 69A.

The draw bolt portion 74F of the draw bolt 72 is inserted through aperture 75A of retractor clamp block 75 and through coaxial fixed jaw block apertures 76D and 76G. Clevis 76Q of moving jaw block 76B is inserted around fixed jaw block hinge point 76N until moving jaw 76K is in alignment with fixed jaw 76J. Draw bolt pin aperture 74H is in horizontal and vertical alignment with coaxial draw bolt pin apertures 76M on moving jaw block 76B. Draw bolt link pin 74G is inserted through apertures 74H and 76M such that draw bolt portion 74F is held between coaxial apertures 76M on moving jaw block 76B. The jaws preferably have an arcuate jaw surface to retain a cylindrical or spherical body.

Moving jaw 76K is hinged at fixed jaw block hinge point 76N, shown in Figure 5B. Pin 76H is inserted through coaxial moving jaw block apertures 76L1 and 76L2, and through hinge point aperture 76P to provide rotation of moving jaw moving jaw block 76B about fixed jaw block 76C, such that when the proximal end of moving jaw block 76B is raised, moving jaw 76K at the distal end of block 76B is lowered to effect clamping, and when said proximal end of block 76B is lowered, jaw 76K is raised.

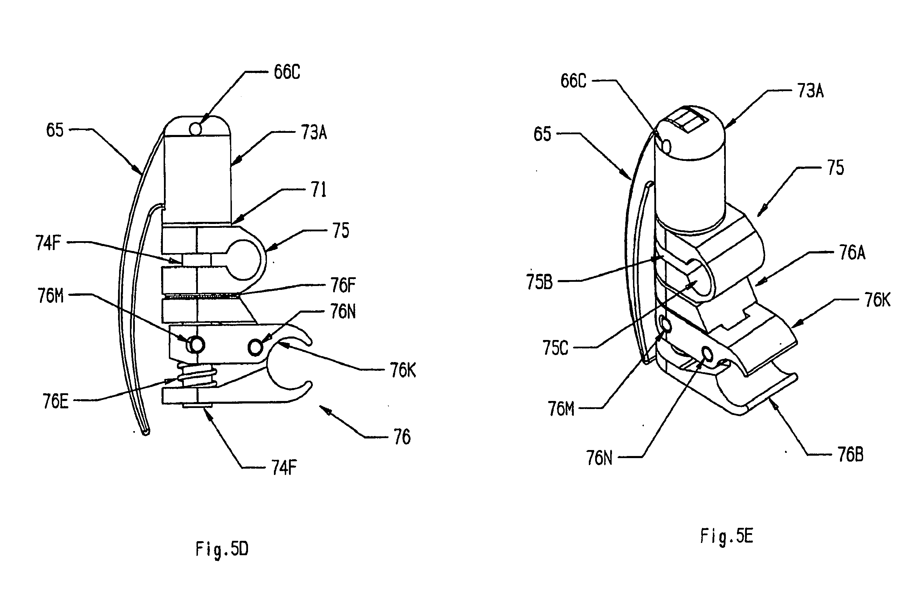

Figures 5B and 5C show retractor clamp 7A in an unclamped position. Figures 5D and 5E show retractor clamp 7A in a clamped position. As lever 65 moves from the unclamped position to the clamped position, intermediate link 69A is pushed into housing 73C at its clevis end 69D by second hinge point 66B of lever 65. This causes the draw bolt/ slide link combination 72 (also referred to as the output link) to be raised vertically and pull pin 74G upward. The vertical, upward force on pin 74G forces the proximal end of moving jaw block 76B upward, which in turn forces moving jaw 76K downward toward fixed jaw 76J, thereby clamping support arm 5. Moving jaw 76K is forced to move downward since moving jaw block 76B is pinioned to draw bolt portion 74F by link pin 74G, which extends through coaxial pin apertures 76M of moving jaw block 76B and pin aperture 74H of draw bolt portion 74F. Accordingly, the output link is operably connected to at least one of the first and second clamp members.

Additionally, retractor clamp block 75 also closes when draw bolt portion 74F is raised. Its clamping force is applied to surgical equipment such as retractor 6 by clamping portion 75C when the diameter of portion 75C is compressed due to squeezing of slot 75B when retractor clamp 7A is in a clamped position. Furthermore, when draw bolt portion 74F is raised, support arm clamp 76 and retractor clamp block 75 are squeezed toward each other, inhibiting rotation of clamp 76 and block 75 with respect to one another

Conversely, when lever 65 is raised to the open position shown in figures 5B and 5C, draw bolt-slide link combination 72 is released downward whereby squeezing of coupling slot 75B and lifting pressure on moving jaw block 76B are relieved. In this manner, retractor 6 and either support arm 5A or 5B are unclamped. Additionally, when lever 65 is raised, squeezing of support arm clamp 76 toward retractor clamp block 75 is relieved; allowing support arm clamp 76 and retractor clamp block 75 to rotate with respect to one another.

Optionally, in a preferred embodiment, spring 76E directly or indirectly moves moving jaw 76K toward fixed jaw 76J when lever 65 is open and clamp 7A is unclamped. In this manner, support arm clamp 76 of retractor clamp 7A acts as a slideable spring clamp around support arm 5A or 5B to assist the operator in sliding retractor clamp 7A in a desired position along support arm 5A or 5B, and to allow for placement of a desired surgical equipment in retractor clamp block 75 in a desired position before closing lever 65 to lock clamp 7A in place.

Optionally, in a highly preferred embodiment, spring 76E lies between the lower part of the proximal end of fixed jaw block 76C and the proximal end of moving jaw block 76B, as illustrated in Figure 5D. Draw bolt portion 74F passes through the center of spring 76E.

In another embodiment friction washer 71 is provided. Friction washer 71 reduces friction between the bottom of draw bolt head 73A and the top surface of clamp block 75 while clamp 7A is unclamped, thus facilitating rotation of retractor clamp block 75 with respect to head 73A until the operator rotates block 75 to the desired orientation and locks it in place by closing lever 65. It is preferred, though not essential, that the outside diameter of friction washer 71 is about the same as the outside diameter of draw bolt head 73A. Its inside diameter is about the same as the outside diameter of draw bolt portion 74F.

In another embodiment, mating radial locking serrations 75F and 76F on the lower portion of clamp block 75 and the upper portion of clamp block 76 respectively, are provided to aid in inhibiting rotation of block 75 with respect to block 76 when lever 65 is closed. Mating radial locking serrations 76F and 75F are, respectively, in axial alignment with base block aperture 76D and clamp block aperture 75A at the bottom of retractor clamp block 75.

Another embodiment of the retractor clamp is assembled in the same manner as coupling clamp 3, illustrated in figures 4A through 4D and described above, with clamp blocks 26 and 27 of suitable size for one clamp block to clamp a support arm 5A while the other clamp block clamps surgical equipment such as a retractor 6. Such a retractor clamp is used in a similar manner to coupling clamp 3, such that an operator slides said retractor clamp onto the distal end of support arm 5A.

The term "connected" refers to both direct or indirect connecting means unless otherwise stated. The terms "rotatable," "rotation," and "pivotally" refer to rotational capability and movement that may be partial or incremental.

Several alternative embodiments and examples have been described and illustrated herein. A person of ordinary skill in the art would appreciate the features of the individual embodiments, and the possible combinations and variations of the components. A person of ordinary skill in the art would further appreciate that any of the embodiments could be provided in any combination with the other embodiments disclosed herein. Additionally, the terms "first," "second," "third," and "fourth" as used herein are intended for illustrative purposes only and do not limit the embodiments in any way. Further, the term "plurality" as used herein indicates any number greater than one, either disjunctively or conjunctively, as necessary, up to an infinite number. Additionally, the term "having" as used herein in both the disclosure and claims, is utilized in an open-ended manner.

It will be understood that the invention may be embodied in other specific forms without departing from the central characteristics thereof. The present examples and embodiments, therefore, are to be considered in all respects as illustrative and not restrictive, and not to be limited to the details given herein.

## Claims

1. A surgical clamp 130 for use with a surgical retractor support frame 1A, the surgical clamp comprising:
a clamp housing 36A, 360A having first and second clamp members connected thereto, the first clamp member 16A, 160A having a first arcuate jaw surface and the first clamp member 16A, 160A being actuated by a first handle 14A, 140A, the second clamp member 16B, 160B having a second arcuate jaw surface and the second clamp member being actuated by a second handle 14B, 140B, **characterised in that** the clamp housing 36A, 360A has a third clamp member contected thereto, the third clamp member 33E having a third arcuate jaw surface and the third clamp member 33E being actuated by a third handle 35, 350, wherein the third clamp member 33E has a clamp axis transverse to a clamp axis of the first 16A, 160A and second clamp members 16B, 160B.

2. The surgical clamp of claim 1, wherein a plane of the clamp axis of the third clamp member 33E is orientated approximately 90° to a plane of the clamp axis of the first 16A, 160A and second clamp members 16B, 160B.

3. The surgical clamp of claim 1 or claim 2, wherein at least one of the first, second and third handles comprises a rotatable handle that rotates about a first pivot point from a first open position to a second closed position, the rotatable handle having a second pivot point eccentric to the first pivot point, and an intermediate link 17A, 17B, 18, 170B having a first portion that rotates about the second pivot point, the intermediate link 17A, 17B, 18, 170B being manipulated by rotation of the handle, and the intermediate link 17A, 17B, 18, 170B further having a second portion that is rotatably connected to a third pivot point on an output link 19A, 132 to manipulate the output link 19A, 132, the output link 19A, 132 being operably connected to the clamp member associated with the at least one of the first, second and third handles to lock the clamp member associated with the at least one of the first, second and third handles.

## Patentansprüche

1. Chirurgische Klemme 130 zur Verwendung mit einem chirurgischen Retraktorhalterahmen 1A, wobei die chirurgische Klemme Folgendes umfasst:
ein Klemmengehäuse 36A, 360A, das ein daran angeschlossenes erstes und ein zweites Klemmelement aufweist, wobei das erste Klemmelement 16A, 160A eine erste bogenförmige Klemmbackenfläche aufweist und das erste Klemmelement 16A, 160A durch einen ersten Griff 14A, 140A betätigt wird, wobei das zweite Klemmelement 16B, 160B eine zweite bogenförmige Klemmbackenfläche aufweist und das zweite Klemmelement durch einen zweiten Griff 14B, 140B betätigt wird, **dadurch gekennzeichnet, dass** das Klemmengehäuse 36A, 360A ein daran angeschlossenes drittes Klemmelement aufweist, wobei das dritte Klemmelement 33E eine dritte bogenförmige Klemmbackenfläche aufweist und das dritte Klemmelement 33E durch einen dritten Griff 35, 350 betätigt wird, wobei das dritte Klemmelement 33E eine Klemmenachse aufweist, die quer zur Klemmenachse des ersten 16A, 160A und des zweiten Klemmelements 16B, 160B liegt.

2. Chirurgische Klemme nach Anspruch 1, wobei eine Ebene der Klemmenachse des dritten Klemmelements 33E ungefähr im 90°-Winkel zu einer Ebene der Klemmenachse des ersten 16A, 160A und des zweiten Klemmelements 16B, 160B ausgerichtet ist.

3. Chirurgische Klemme nach Anspruch 1 oder 2, wobei der erste und/oder der zweite und/oder der dritte Griff einen drehbaren Griff umfasst, der sich um einen ersten Drehpunkt aus einer ersten, geöffneten Position in eine zweite, geschlossene Position dreht, wobei der drehbare Griff einen zweiten, zum ersten Drehpunkt exzentrisch gelegenen Drehpunkt und eine Zwischenverbindung 17A, 17B, 18, 170B mit einem ersten Abschnitt aufweist, der sich um den zweiten Drehpunkt dreht, wobei die Zwischenverbindung 17A, 17B, 18, 170B durch Drehung des Griffs bedient wird, wobei die Zwischenverbindung 17A, 17B, 18, 170B des Weiteren einen zweiten Abschnitt aufweist, der drehbar an einen dritten Drehpunkt an einer Ausgabeverbindung 19A, 132 angeschlossen ist, um die Ausgabeverbindung 19A, 132 zu bedienen, wobei die Ausgabeverbindung 19A, 132 funktionsfähig an das Klemmelement angeschlossen ist, das zum ersten und/oder zweiten und/oder dritten Griff gehört, um das Klemmelement, das zum ersten und/oder zweiten und/oder dritten Griff gehört, zu arretieren.

## Revendications

1. Pince chirurgical 130 destinée à être utilisée avec une armature de support d'écarteur chirurgical 1A, la pince chirurgicale comprenant :
un boitier de pince 36A, 360A auquel sont reliés des premier et deuxième organes de pince, le premier organe de pince 16A, 160A ayant une première surface de mâchoire arquée et le premier organe de pince 16A, 160A étant actionné par une première poignée 14A, 140A, le deuxième organe de pince 16B, 160B ayant une deuxième surface de mâchoire arquée et le deuxième organe de pince étant actionné par une deuxième poignée 14B, 140B, **caractérisée en ce que** le boîtier de pince 36A, 360A est relié à un troisième organe de pince, le troisième organe de pince 33E ayant une troisième surface de mâchoire arquée et le troisième organe de pince 33E étant actionné par une troisième poignée 35, 350, dans laquelle le troisième organe de poignée 33E a un axe de pince transversal à un axe de pince des premier 16A, 160A et deuxième organes de pince 16B, 160B.

2. Pince chirurgicale selon la revendication 1, dans laquelle un plan de l'axe de pince du troisième organe de pince 33E est orienté approximativement à 90° par rapport à un plan de l'axe de pince des premier 16A, 160A et deuxième organes de pince 16B, 160B.

3. Pince chirurgicale selon la revendication 1 ou la revendication 2, dans laquelle au moins une des première, deuxième et troisième poignées comprend une poignée rotative qui tourne autour d'un premier point de pivot d'une première position ouverte à une seconde position fermée, la poignée rotative ayant un second point pivot excentrique au premier point de pivot, et une liaison intermédiaire 17A, 17B, 18, 170B ayant une première partie qui tourne autour du deuxième point de pivot, la liaison intermédiaire 17A, 17B, 18, 170B étant manipulée par rotation de la poignée, et la liaison intermédiaire 17A, 17B, 18, 170B ayant en outre une seconde partie qui est reliée en rotation à un troisième point de pivot sur une liaison de sortie 19A, 132 pour manipuler la liaison de sortie 19A, 132, la liaison de sortie 19A, 132 étant reliée opérationnellement à l'organe de pince associé à au moins l'une des première, deuxième et troisième poignées pour verrouiller l'organe de pince associé à au moins l'une des première, deuxième et troisième poignées.
